# EUROPEAN PATENT SPECIFICATION

(11) **EP 2 649 093 B1**
(45) Date of publication and mention of the grant of the patent: **10.08.2016**
(21) Application number: 11807881.5
(22) Date of filing: 12.12.2011
(51) Int. Cl.: C07K 14/47, G01N 33/564

(54) **BIOMARKERS FOR THE DIAGNOSIS OF MULTIPLE SCLEROSIS**
BIOMARKER ZUR DIAGNOSE VON MULTIPLER SKLEROSE
BIOMARQUEURS POUR LE DIAGNOSTIC DE LA SCLÉROSE EN PLAQUES

(30) Priority: 10.12.2010 IT PD20100374
(43) Date of publication of application: 16.10.2013
(73) Proprietor: Universita Degli Studi di Trieste, 34127 Trieste (IT)
(72) Inventor: EDOMI, Paolo, 34127 Trieste (IT); BEMBICH, Sara, 34128 Trieste (IT); CORTINI, Andrea, 33052 Cervignano del Friuli (IT)
(74) Representative: Zaccaro, Elisabetta
(86) International application number: PCT/EP2011/072440
(87) International publication number: WO 2012/076722

(56) References cited:
- WO-A1-02/079218
- OTA K ET AL: "T-CELL RECOGNITION OF AN IMMUNODOMINANT MYELIN BASIC PROTEIN EPITOPE IN MULTIPLE SCLEROSIS", NATURE: INTERNATIONAL WEEKLY JOURNAL OF SCIENCE, NATURE PUBLISHING GROUP, UNITED KINGDOM, vol. 346, no. 6280, 12 July 1990 (1990-07-12), pages 183-187, XP000652362, ISSN: 0028-0836, DOI: 10.1038/346183A0
- BRUNO GRAN ET AL: "T Cells, Cytokines, and Autoantigens in Multiple Sclerosis", CURRENT NEUROLOGY AND NEUROSCIENCE REPORTS, vol. 1, no. 3, 1 May 2001 (2001-05-01), pages 263-270, XP55019880,
- HOLBERT S ET AL: "The Gln-Ala repeat transcriptional activator CA150 interacts with huntingtin: neuropathologic and genetic evidence for a role in Huntington's disease pathogenesis", PROCEEDINGS OF THE NATIONAL ACADEMY OF SCIENCES, NATIONAL ACADEMY OF SCIENCES, WASHINGTON, DC; US, vol. 98, no. 4, 13 February 2001 (2001-02-13), pages 1811-1816, XP002610882, ISSN: 0027-8424, DOI: DOI:10.1073/PNAS.041566798 [retrieved on 2001-01-30]
- Andrea Cortini: "Serum profiling and autoantibodies identification in Multiple Sclerosis using epitope and CSF IgG phage display libraries", , 30 April 2009 (2009-04-30), XP55019891, Retrieved from the Internet: URL:http://hdl.handle.net/10077/3073 [retrieved on 2012-02-21]
- ERIKA MALINI ET AL: "HMGA Interactome: New Insights from Phage Display Technology", BIOCHEMISTRY, vol. 50, no. 17, 3 May 2011 (2011-05-03), pages 3462-3468, XP55019886, ISSN: 0006-2960, DOI: 10.1021/bi200101f
- Uniparc: "UPI0001586CBE", , 15 January 2010 (2010-01-15), XP055111647, [retrieved on 2014-04-02]

### SEQUENCE LISTING

<110> UNIVERSITA' DEGLI STUDI DI TRIESTE
<120> BIOMARKERS FOR THE DIAGNOSIS OF MULTIPLE SCLEROSIS
<130> 10440PTWO
<150> ITPD2010A000374
   <151> 2010-12-10
<160> 6
<170> BiSSAP 1.0
<210> 1
   <211> 1098
   <212> PRT
   <213> Homo sapiens
<220>
   <221> SOURCE
   <222> 1..1098
   <223> /mol_type="protein"
   /organism="Homo sapiens"
<400> 1
   Met Ala Glu Arg Gly Gly Asp Gly Gly Glu Ser Glu Arg Phe Asn Pro
   1               5                   10                  15
   Gly Glu Leu Arg Met Ala Gln Gln Gln Ala Leu Arg Phe Arg Gly Pro
               20                  25                  30
   Ala Pro Pro Pro Asn Ala Val Met Arg Gly Pro Pro Pro Leu Met Arg
           35                  40                  45
   Pro Pro Pro Pro Phe Gly Met Met Arg Gly Pro Pro Pro Pro Pro Arg
       50                  55                  60
   Pro Pro Phe Gly Arg Pro Pro Phe Asp Pro Asn Met Pro Pro Met Pro
   65                  70                  75                  80
   Pro Pro Gly Gly Ile Pro Pro Pro Met Gly Pro Pro His Leu Gln Arg
                   85                  90                  95
   Pro Pro Phe Met Pro Pro Pro Met Ser Ser Met Pro Pro Pro Pro Gly
               100                 105                 110
   Met Met Phe Pro Pro Gly Met Pro Pro Val Thr Ala Pro Gly Thr Pro
           115                 120                 125
   Ala Leu Pro Pro Thr Glu Glu Ile Trp Val Glu Asn Lys Thr Pro Asp
       130                 135                 140
   Gly Lys Val Tyr Tyr Tyr Asn Ala Arg Thr Arg Glu Ser Ala Trp Thr
   145                 150                 155                 160
   Lys Pro Asp Gly Val Lys Val Ile Gln Gln Ser Glu Leu Thr Pro Met
                   165                 170                 175
   Leu Ala Ala Gln Ala Gln Val Gln Ala Gln Ala Gln Ala Gln Ala Gln
               180                 185                 190
   Ala Gln Ala Gln Ala Gln Ala Gln Ala Gln Ala Gln Ala Gln Ala Gln
           195                 200                 205
   Ala Gln Ala Gln Ala Gln Ala Gln Ala Gln Ala Gln Ala Gln Ala Gln
       210                 215                 220
   Ala Gln Ala Gln Ala Gln Ala Gln Ala Gln Ala Gln Ala Gln Ala Gln
   225                 230                 235                 240
   Ala Gln Val Gln Ala Gln Val Gln Ala Gln Val Gln Ala Gln Ala Val
                   245                 250                 255
   Gly Ala Ser Thr Pro Thr Thr Ser Ser Pro Ala Pro Ala Val Ser Thr
               260                 265                 270
   Ser Thr Ser Ser Ser Thr Pro Ser Ser Thr Thr Ser Thr Thr Thr Thr
           275                 280                 285
   Ala Thr Ser Val Ala Gln Thr Val Ser Thr Pro Thr Thr Gln Asp Gln
       290                 295                 300
   Thr Pro Ser Ser Ala Val Ser Val Ala Thr Pro Thr Val Ser Val Ser
   305                 310                 315                 320
   Thr Pro Ala Pro Thr Ala Thr Pro Val Gln Thr Val Pro Gln Pro His
                   325                 330                 335
   Pro Gln Thr Leu Pro Pro Ala Val Pro His Ser Val Pro Gln Pro Thr
               340                 345                 350
   Thr Ala Ile Pro Ala Phe Pro Pro Val Met Val Pro Pro Phe Arg Val
           355                 360                 365
   Pro Leu Pro Gly Met Pro Ile Pro Leu Pro Gly Val Ala Met Met Gln
       370                 375                 380
   Ile Val Ser Cys Pro Tyr Val Lys Thr Val Ala Thr Thr Lys Thr Gly
   385                 390                 395                 400
   Val Leu Pro Gly Met Ala Pro Pro Ile Val Pro Met Ile His Pro Gln
                   405                 410                 415
   Val Ala Ile Ala Ala Ser Pro Ala Thr Leu Ala Gly Ala Thr Ala Val
               420                 425                 430
   Ser Glu Trp Thr Glu Tyr Lys Thr Ala Asp Gly Lys Thr Tyr Tyr Tyr
           435                 440                 445
   Asn Asn Arg Thr Leu Glu Ser Thr Trp Glu Lys Pro Gln Glu Leu Lys
       450                 455                 460
   Glu Lys Glu Lys Leu Glu Glu Lys Ile Lys Glu Pro Ile Lys Glu Pro
   465                 470                 475                 480
   Ser Glu Glu Pro Leu Pro Met Glu Thr Glu Glu Glu Asp Pro Lys Glu
                   485                 490                 495
   Glu Pro Ile Lys Glu Ile Lys Glu Glu Pro Lys Glu Glu Glu Met Thr
               500                 505                 510
   Glu Glu Glu Lys Ala Ala Gln Lys Ala Lys Pro Val Ala Thr Ala Pro
           515                 520                 525
   Ile Pro Gly Thr Pro Trp Cys Val Val Trp Thr Gly Asp Glu Arg Val
       530                 535                 540
   Phe Phe Tyr Asn Pro Thr Thr Arg Leu Ser Met Trp Asp Arg Pro Asp
   545                 550                 555                 560
   Asp Leu Ile Gly Arg Ala Asp Val Asp Lys Ile Ile Gln Glu Pro Pro
                   565                 570                 575
   His Lys Lys Gly Met Glu Glu Leu Lys Lys Leu Arg His Pro Thr Pro
               580                 585                 590
   Thr Met Leu Ser Ile Gln Lys Trp Gln Phe Ser Met Ser Ala Ile Lys
           595                 600                 605
   Glu Glu Gln Glu Leu Met Glu Glu Ile Asn Glu Asp Glu Pro Val Lys
       610                 615                 620
   Ala Lys Lys Arg Lys Arg Asp Asp Asn Lys Asp Ile Asp Ser Glu Lys
   625                 630                 635                 640
   Glu Ala Ala Met Glu Ala Glu Ile Lys Ala Ala Arg Glu Arg Ala Ile
                   645                 650                 655
   Val Pro Leu Glu Ala Arg Met Lys Gln Phe Lys Asp Met Leu Leu Glu
               660                 665                 670
   Arg Gly Val Ser Ala Phe Ser Thr Trp Glu Lys Glu Leu His Lys Ile
           675                 680                 685
   Val Phe Asp Pro Arg Tyr Leu Leu Leu Asn Pro Lys Glu Arg Lys Gln
       690                 695                 700
   Val Phe Asp Gln Tyr Val Lys Thr Arg Ala Glu Glu Glu Arg Arg Glu
   705                 710                 715                 720
   Lys Lys Asn Lys Ile Met Gln Ala Lys Glu Asp Phe Lys Lys Met Met
                   725                 730                 735
   Glu Glu Ala Lys Phe Asn Pro Arg Ala Thr Phe Ser Glu Phe Ala Ala
               740                 745                 750
   Lys His Ala Lys Asp Ser Arg Phe Lys Ala Ile Glu Lys Met Lys Asp
           755                 760                 765
   Arg Glu Ala Leu Phe Asn Glu Phe Val Ala Ala Ala Arg Lys Lys Glu
       770                 775                 780
   Lys Glu Asp Ser Lys Thr Arg Gly Glu Lys Ile Lys Ser Asp Phe Phe
   785                 790                 795                 800
   Glu Leu Leu Ser Asn His His Leu Asp Ser Gln Ser Arg Trp Ser Lys
                   805                 810                 815
   Val Lys Asp Lys Val Glu Ser Asp Pro Arg Tyr Lys Ala Val Asp Ser
               820                 825                 830
   Ser Ser Met Arg Glu Asp Leu Phe Lys Gln Tyr Ile Glu Lys Ile Ala
           835                 840                 845
   Lys Asn Leu Asp Ser Glu Lys Glu Lys Glu Leu Glu Arg Gln Ala Arg
       850                 855                 860
   Ile Glu Ala Ser Leu Arg Glu Arg Glu Arg Glu Val Gln Lys Ala Arg
   865                 870                 875                 880
   Ser Glu Gln Thr Lys Glu Ile Asp Arg Glu Arg Glu Gln His Lys Arg
                   885                 890                 895
   Glu Glu Ala Ile Gln Asn Phe Lys Ala Leu Leu Ser Asp Met Val Arg
               900                 905                 910
   Ser Ser Asp Val Ser Trp Ser Asp Thr Arg Arg Thr Leu Arg Lys Asp
           915                 920                 925
   His Arg Trp Glu Ser Gly Ser Leu Leu Glu Arg Glu Glu Lys Glu Lys
       930                 935                 940
   Leu Phe Asn Glu His Ile Glu Ala Leu Thr Lys Lys Lys Arg Glu His
   945                 950                 955                 960
   Phe Arg Gln Leu Leu Asp Glu Thr Ser Ala Ile Thr Leu Thr Ser Thr
                   965                 970                 975
   Trp Lys Glu Val Lys Lys Ile Ile Lys Glu Asp Pro Arg Cys Ile Lys
               980                 985                 990
   Phe Ser Ser Ser Asp Arg Lys Lys Gln Arg Glu Phe Glu Glu Tyr Ile
           995                 1000                1005
   Arg Asp Lys Tyr Ile Thr Ala Lys Ala Asp Phe Arg Thr Leu Leu Lys
       1010                1015                1020
   Glu Thr Lys Phe Ile Thr Tyr Arg Ser Lys Lys Leu Ile Gln Glu Ser
   1025                1030                1035                1040
   Asp Gln His Leu Lys Asp Val Glu Lys Ile Leu Gln Asn Asp Lys Arg
                   1045                1050                1055
   Tyr Leu Val Leu Asp Cys Val Pro Glu Glu Arg Arg Lys Leu Ile Val
               1060                1065                1070
   Ala Tyr Val Asp Asp Leu Asp Arg Arg Gly Pro Pro Pro Pro Pro Thr
           1075                1080                1085
   Ala Ser Glu Pro Thr Arg Arg Ser Thr Lys
       1090                1095
<210> 2
   <211> 422
   <212> PRT
   <213> Homo sapiens
<220>
   <221> SOURCE
   <222> 1..422
   <223> /mol_type="protein"
   /organism="Homo sapiens"
<400> 2
   Ala Phe Ser Thr Trp Glu Lys Glu Leu His Lys Ile Val Phe Asp Pro
   1               5                   10                  15
   Arg Tyr Leu Leu Leu Asn Pro Lys Glu Arg Lys Gln Val Phe Asp Gln
               20                  25                  30
   Tyr Val Lys Thr Arg Ala Glu Glu Glu Arg Arg Glu Lys Lys Asn Lys
           35                  40                  45
   Ile Met Gln Ala Lys Glu Asp Phe Lys Lys Met Met Glu Glu Ala Lys
       50                  55                  60
   Phe Asn Pro Arg Ala Thr Phe Ser Glu Phe Ala Ala Lys His Ala Lys
   65                  70                  75                  80
   Asp Ser Arg Phe Lys Ala Ile Glu Lys Met Lys Asp Arg Glu Ala Leu
                   85                  90                  95
   Phe Asn Glu Phe Val Ala Ala Ala Arg Lys Lys Glu Lys Glu Asp Ser
               100                 105                 110
   Lys Thr Arg Gly Glu Lys Ile Lys Ser Asp Phe Phe Glu Leu Leu Ser
           115                 120                 125
   Asn His His Leu Asp Ser Gln Ser Arg Trp Ser Lys Val Lys Asp Lys
       130                 135                 140
   Val Glu Ser Asp Pro Arg Tyr Lys Ala Val Asp Ser Ser Ser Met Arg
   145                 150                 155                 160
   Glu Asp Leu Phe Lys Gln Tyr Ile Glu Lys Ile Ala Lys Asn Leu Asp
                   165                 170                 175
   Ser Glu Lys Glu Lys Glu Leu Glu Arg Gln Ala Arg Ile Glu Ala Ser
               180                 185                 190
   Leu Arg Glu Arg Glu Arg Glu Val Gln Lys Ala Arg Ser Glu Gln Thr
           195                 200                 205
   Lys Glu Ile Asp Arg Glu Arg Glu Gln His Lys Arg Glu Glu Ala Ile
       210                 215                 220
   Gln Asn Phe Lys Ala Leu Leu Ser Asp Met Val Arg Ser Ser Asp Val
   225                 230                 235                 240
   Ser Trp Ser Asp Thr Arg Arg Thr Leu Arg Lys Asp His Arg Trp Glu
                   245                 250                 255
   Ser Gly Ser Leu Leu Glu Arg Glu Glu Lys Glu Lys Leu Phe Asn Glu
               260                 265                 270
   His Ile Glu Ala Leu Thr Lys Lys Lys Arg Glu His Phe Arg Gln Leu
           275                 280                 285
   Leu Asp Glu Thr Ser Ala Ile Thr Leu Thr Ser Thr Trp Lys Glu Val
       290                 295                 300
   Lys Lys Ile Ile Lys Glu Asp Pro Arg Cys Ile Lys Phe Ser Ser Ser
   305                 310                 315                 320
   Asp Arg Lys Lys Gln Arg Glu Phe Glu Glu Tyr Ile Arg Asp Lys Tyr
                   325                 330                 335
   Ile Thr Ala Lys Ala Asp Phe Arg Thr Leu Leu Lys Glu Thr Lys Phe
               340                 345                 350
   Ile Thr Tyr Arg Ser Lys Lys Leu Ile Gln Glu Ser Asp Gln His Leu
           355                 360                 365
   Lys Asp Val Glu Lys Ile Leu Gln Asn Asp Lys Arg Tyr Leu Val Leu
       370                 375                 380
   Asp Cys Val Pro Glu Glu Arg Arg Lys Leu Ile Val Ala Tyr Val Asp
   385                 390                 395                 400
   Asp Leu Asp Arg Arg Gly Pro Pro Pro Pro Pro Thr Ala Ser Glu Pro
                   405                 410                 415
   Thr Arg Arg Ser Thr Lys
               420
<210> 3
   <211> 111
   <212> PRT
   <213> Homo sapiens
<220>
   <221> SOURCE
   <222> 1..111
   <223> /mol_type="protein"
   /organism="Homo sapiens"
<400> 3
   Lys Glu Leu His Lys Ile Val Phe Asp Pro Arg Tyr Leu Leu Leu Asn
   1               5                   10                  15
   Pro Lys Glu Arg Lys Gln Val Phe Asp Gln Tyr Val Lys Thr Arg Ala
               20                  25                  30
   Glu Glu Glu Arg Arg Glu Lys Lys Asn Lys Ile Met Gln Ala Lys Glu
           35                  40                  45
   Asp Phe Lys Lys Met Met Glu Glu Ala Lys Phe Asn Pro Arg Ala Thr
       50                  55                  60
   Phe Ser Glu Phe Ala Ala Lys His Ala Lys Asp Ser Arg Phe Lys Ala
   65                  70                  75                  80
   Ile Glu Lys Met Lys Asp Arg Glu Ala Leu Phe Asn Glu Phe Val Ala
                   85                  90                  95
   Ala Ala Arg Lys Lys Glu Lys Glu Asp Ser Lys Thr Arg Gly Glu
               100                 105                 110
<210> 4
   <211> 20
   <212> PRT
   <213> Homo sapiens
<220>
   <221> SOURCE
   <222> 1..20
   <223> /mol_type="protein"
   /organism="Homo sapiens"
<400> 4
   Ala Ala Lys His Ala Lys Asp Ser Arg Phe Lys Ala Ile Glu Lys Met
   1               5                   10                  15
   Lys Asp Arg Glu
               20
<210> 5
   <211> 23
   <212> PRT
   <213> Homo sapiens
<220>
   <221> SOURCE
   <222> 1..23
   <223> /mol_type="protein"
   /organism="Homo sapiens"
<400> 5
   Asp Gln Tyr Val Lys Thr Arg Ala Glu Glu Glu Arg Arg Glu Lys Lys
   1               5                   10                  15
   Asn Lys Ile Met Gln Ala Lys
               20
<210> 6
   <211> 21
   <212> PRT
   <213> Homo sapiens
<220>
   <221> SOURCE
   <222> 1..21
   <223> /mol_type="protein"
   /organism="Homo sapiens"
<400> 6
   Glu Asp Phe Lys Lys Met Met Glu Glu Ala Lys Phe Asn Pro Arg Ala
   1               5                   10                  15
   Thr Phe Ser Glu Phe
               20

## Description

### Field of the invention

The invention relates to a protein consisting of Seq ID N° 2 as biological marker for use in the diagnosis of multiple sclerosis and the use of said marker for distinguishing between patients with multiple sclerosis and patients with other neurological diseases. The invention further relates to a diagnostic technique for multiple sclerosis using said biological marker.

### Prior art

Multiple sclerosis (MS) is regarded as the prototype of inflammatory autoimmune diseases of the central nervous system (CNS). MS is the commonest neurological pathology in young adults, affecting more than a million individuals world-wide, including 400,000 in Europe and nearly 60,000 in Italy, with highest incidence in Sardinia (1 in 700 inhabitants) and causes substantial invalidity of 50% of patients. The characteristic feature of the disease is demyelination plaque. It is known that the development of the plaque of the lesion involves an initial inflammatory phase, followed by a progressive chronic phase, and although the individual stages have not yet been fully elucidated, most of the evidence is in favour of an autoimmune pathogenesis. The factor that triggers the disease is still unknown, but the most widely held view is that the autoimmune reaction depends on a new contact with environmental factors, such as viruses, which mimic molecules of self (molecular mimicry) (Svejgaard et al., 2008).

While the role of the T cells in the pathogenesis of MS is well known, the role of the B cells and autoantibodies is largely unsolved, although their importance is becoming more and more evident (Klawiter and Cross, 2007). At the same time, the importance of identifying biological markers of MS is growing steadily, especially because of the heterogeneity of patients' immune response (Reindl et al., 2006).

There are various indications that there is a link between antibodies, both of class G and of class M, and the disease. B cells and specific myelin autoantibodies are present in the plaques of patients with MS, and an increase in the production of immunoglobulins (Ig) in the cerebrospinal fluid has been observed in more than 90% of patients with MS. More recently, the presence of structures similar to lymphatic follicles has been observed in the meninges of some patients with MS and this has been linked to more acute demyelination (Magliozzi et al., 2007). Further complications result from the phenomenon of "epitope spreading", by which the autoimmune response spreads from the single initial component of an affected tissue to other autoantigens. The progression of murine experimental autoimmune encephalomyelitis (EAE) and of MS is accompanied by a decline in autoreactivity of the primary T cells and concomitant appearance of new autoreactivity simultaneous with the tissue damage mediated by the autoimmune response (McMahon et al., 2005).

Ota et al. disclose myelin basic protein fragments which serve as autoantigen biomarkers suitable for diagnosing MS. The specificity of the epitopes recognized by the anti-myelin antibodies in MS is still an open question. Various epitopes of myelin proteins have occasionally been identified as immunodominant in patients with MS with relapses and remissions (Khalil et al., 2006).

This variability might indicate that identification of the autoantigens involved in triggering or perpetuating MS may largely depend on the system with which they are sought. Whereas all the epitopes of the T cells are linear, an autoantibody specific to myelin proteins can bind a conformational or linear epitope (Dharmasaroja, 2003). Antibodies, especially IgG, that bind conformational epitopes of the extracellular domain of MOG with high affinity were found in patients' serum. However, these pathogenic antibodies have not yet been characterized in humans (Lalive et al., 2006).

Determination of the specificity of the epitopes is of considerable diagnostic value. Whereas the data from conventional magnetic resonance enable clinicians to identify the disease and the particular phase, there are no accepted biological indicators for diagnosing the activity of the disease in MS (Salzer et al., 2010). Therefore there is still a need for identifying a biological marker that is of diagnostic value for multiple sclerosis.

Berger et al. (2003) showed that patients with the clinically isolated form of MS (CIS), seropositive for anti-MOG and anti-MBP antibodies, have a higher probability of having relapses compared with seronegative patients. Moreover, it was found that the titre of IgG, but not of IgM, specific to the native form of MOG, was significantly higher in patients with MS compared with a control group, with a higher prevalence for the patients with the primary progressive form (Zhou et al., 2006).

Conversely, Lim et al. (2005) showed for example that the levels of anti-myelin IgG are not correlated with the clinical parameters of the disease.

Using a cellular assay that measures in detail the antibodies directed against the human MOG protein expressed on the cell membrane, native IgGs specific to MOG were found more frequently in the serum of CIS and RR patients, only marginally in secondary progressive MS and not at all in primary progressive MS (Lalive et al., 2006). However, another study did not find any link between the presence of IgG and IgM anti-MOG and anti-MBP antibodies, detected by Western blot, and progression to clinically defined MS or a diagnosis of MS according to McDonald's criteria (Kuhle et al., 2007). Therefore the diagnostic value of serum antibodies to MOG and MBP for predicting a risk of progression to clinically defined MS in patients who have presented a clinically isolated syndrome is debatable at present (Polman and Killestein, 2007).

In the diagnosis of multiple sclerosis, it will be considered, in particular, that the main interest focuses on the possibility of discriminating between patients with neurological symptoms of various kinds, among various diseases affecting the nervous system, and patients with multiple sclerosis. Multiple sclerosis in fact has a multiplicity of symptoms that can be confused with those of other neurological diseases and sometimes diagnosis by means of nuclear magnetic resonance, the only system currently used, is not definitive (Ratchford and Calabresi, 2008). Consequently there is a need to identify markers that make it possible in particular to distinguish between patients with neurological symptoms, rather than between patients with multiple sclerosis and healthy subjects.

The aim of the present invention is therefore to identify a biological marker that would be of diagnostic value for multiple sclerosis and the use of said markers for distinguishing between patients with multiple sclerosis and patients with other neurological diseases.

A further aim is to identify an innovative system for searching for autoantigens in autoimmune diseases, and application thereof in the case of multiple sclerosis.

### Summary of the invention

The aforementioned aim was achieved with a protein or polypeptide consisting of SEQ ID NO.2 for use as biological marker for the diagnosis of multiple sclerosis. Another aspect of the invention relates to a method for the diagnosis of multiple sclerosis using said protein. A further aspect of the present invention relates to a diagnostic kit for the diagnosis of an autoimmune disease comprising said protein that acts as antigen and a reagent for separate, simultaneous and successive use as a conjugated secondary antibody able to detect the antibodies that are present in biological samples and are reactive towards the protein or antigen used.

### Brief description of the drawings

The characteristics and advantages of the present invention will now be described in detail, referring to the appended Figs. 1-9, and to the non-limiting examples supplied for purposes of illustration.
Fig. 1 a: Schematic representation of the map of the vector pEP1 and the relevant sequence of the polylinker.
Fig. 1b: Schematic representation of the map of the vector pEP2 and the relevant sequence of the polylinker.
Fig. 2: Diagram representing the procedural steps in construction of the antigen phage library indicating the oligonucleotides used.

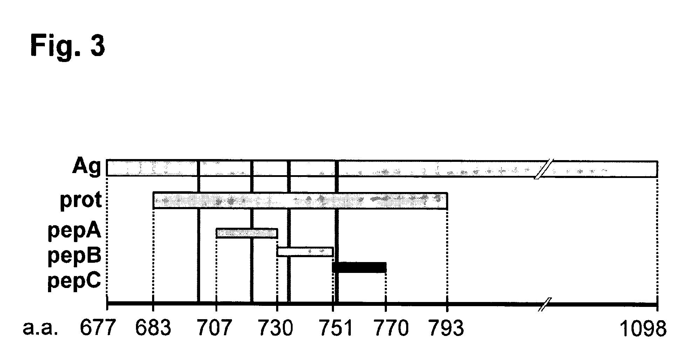

Fig. 3: Schematic representation of the relative positions, with respect to the antigen identified by selection of the phage library: the human TCERG1 protein SEQ ID NO.1 (Ag), of the recombinant protein: the polypeptide that corresponds to the portion between amino acids 677 and 1098 of the human TCERG1 protein SEQ ID NO. 2 (prot) and of the three peptides synthesized for validation of the respective diagnostic potentialities: polypeptide TCERG1A SEQ ID NO. 5 (pepA), polypeptide TCERG1 B SEQ ID NO.6 (pepB) and polypeptide TCERG1 C SEQ ID NO.4 (pepC); the numbers indicated on the line underneath refer to the amino acids of the whole TCERG protein; the vertical bars indicate position of possible epitopes identified by homology with another antigen.
Fig. 4: Electrophoretic analysis of production of the recombinant protein TCERG1 (a.a. 677-1098) by acrylamide gel at 12.5% and Coomassie staining.
Fig. 4 (A): bacterial lysates of cells uninduced (lane 2) and induced for 3 (lane 3) and 16 hours (lane 4).
Fig. 4 (B): samples eluted after passage through column of streptactin: successive elution fractions (lanes from 2 to 5)
   In lane 1: Molecular weight standards (Mark12, Invitrogen).
Fig. 4 (C): samples eluted after passage through column of histidines (C): successive elution fractions (lanes from 2 to 5).
   In lane 1: Molecular weight standards (Mark12, Invitrogen).
Fig. 5: (A) "Scatter plot" representation of the reactivity of CSF samples from 17 patients with multiple sclerosis (MS) and 17 control subjects (OND) in comparison with the TCERG1 antigen carried by phage assayed by ELISA. The ratios of the values of optical density, relative to those obtained in the absence of antigen for each sample, are shown on the ordinate.

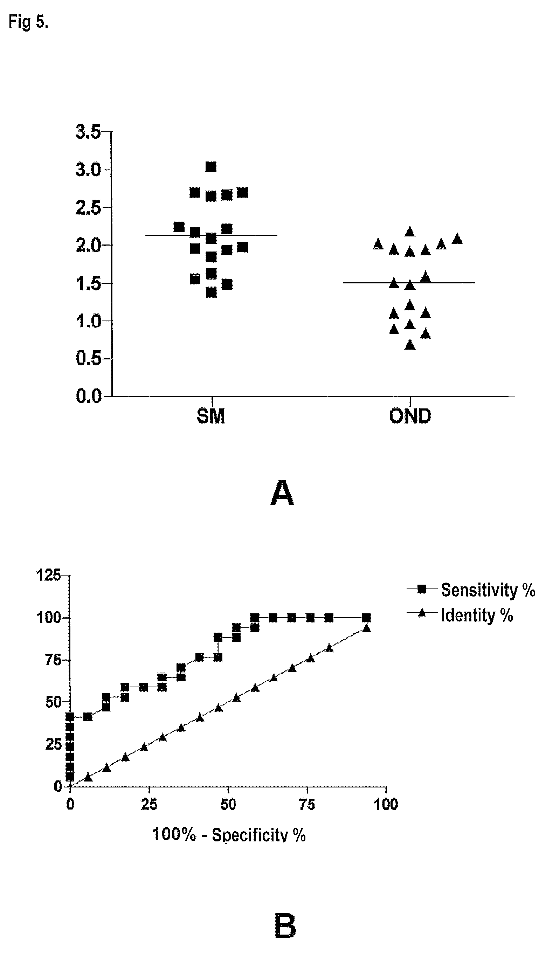

Fig. 5: (B) "Receiver Operating Characteristic" (ROC) graph for determining the diagnostic value of the antigen used in (A).

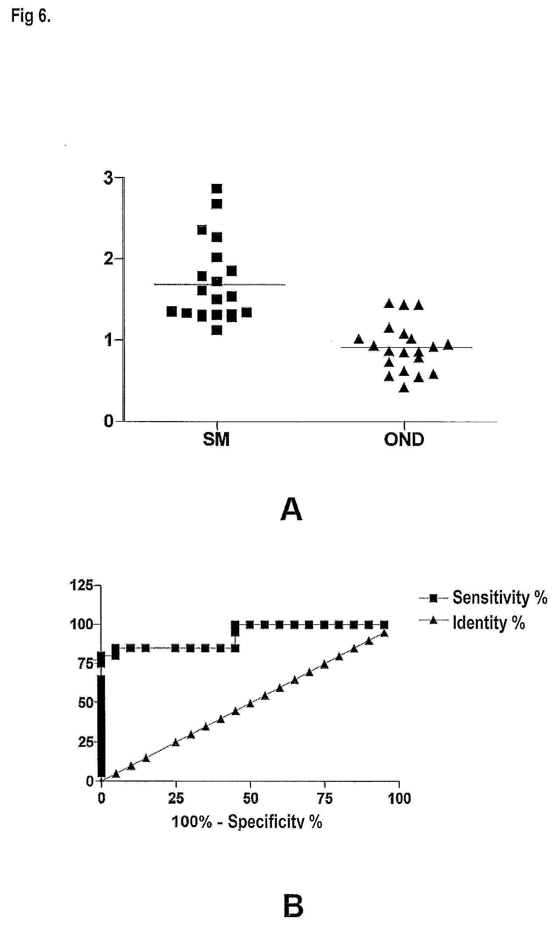

Fig. 6: (A) "Scatter plot" representation of the reactivity of serum samples from 20 patients with multiple sclerosis (MS) and 20 control subjects (OND) in comparison with the antigen TCERG1 as recombinant protein assayed by ELISA. The ratios of the optical density values relative to those obtained in the absence of antigen for each sample are shown on the ordinate.
Fig. 6: (B) ROC graph for determining the diagnostic value of the antigen used in (A).

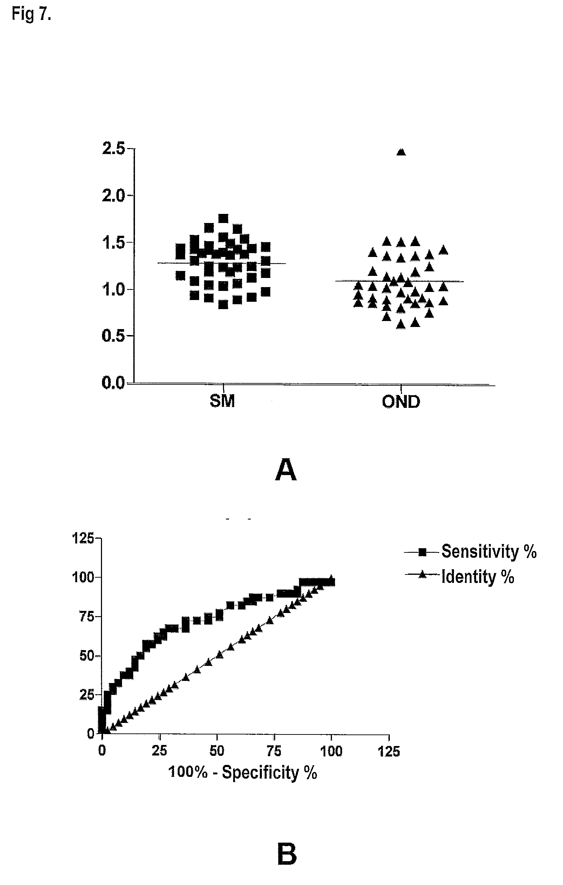

Fig. 7: (A) "Scatter plot" representation of the reactivity of serum samples from 41 patients with multiple sclerosis (MS) and 40 control subjects (OND) in comparison with the peptide TCERG1-C assayed by ELISA. The ratios of the optical density values relative to those obtained in the absence of antigen for each sample are shown on the ordinate.
Fig. 7: (B) ROC graph for determining the diagnostic value of the antigen used in (A).

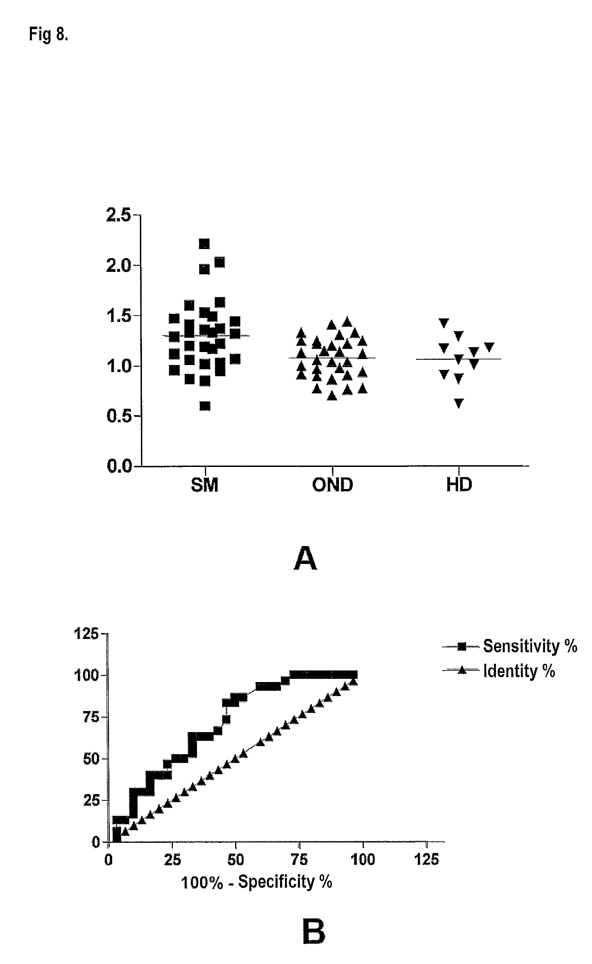

Fig. 8: (A) "Scatter plot" representation of the reactivity of serum samples from 30 patients with multiple sclerosis (MS), 40 control subjects (OND) and 10 healthy subjects (HD) in comparison with the peptide TCERG1-C assayed by ELISA. The ratios of the optical density values relative to those obtained in the absence of antigen for each sample are shown on the ordinate.
Fig. 8: (B) ROC graph for determining the diagnostic value of the antigen used in (A) relative to the MS and OND samples.

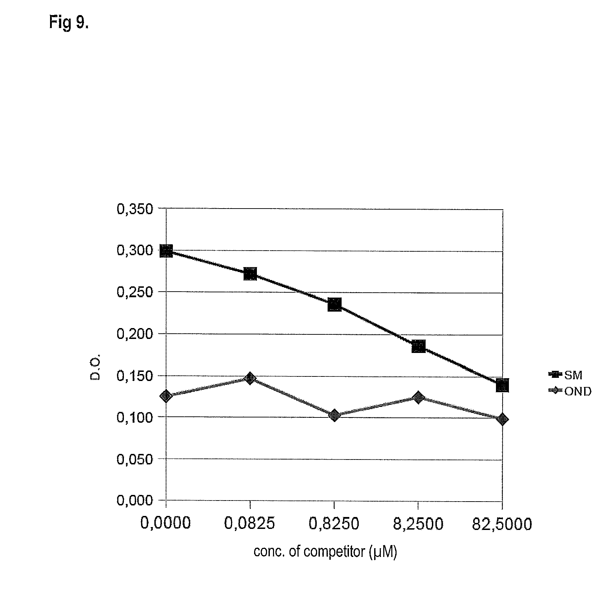

Fig. 9: Representation of competitive ELISA for recognition of the peptide TCERG1-C by a serum sample of a patient with multiple sclerosis and of a control subject. The samples were pre-incubated with increasing concentrations of peptide (µM, on the abscissa) and then assayed by ELISA; the values of optical density are given for each sample, the value of the negative control (absence of antigen) being equal for both sera (symbols: square = MS sample; diamond = control sample).

### Detailed description of the invention

The invention therefore relates to a protein or polypeptide consisting of SEQ ID NO.2 for use as biological marker (or biomarker) for the diagnosis of multiple sclerosis.

For the purposes of the present invention, each protein or polypeptide corresponds to a sequence identified with a sequence number or SEQ ID NO. as given below:
SEQ ID NO. 1 corresponds to the amino acid sequence of the human TCERG1 protein TCERG1 transcription elongation regulator 1; Gene ID: 10915, NM_006706.3→NP_006697.2 transcription elongation regulator 1 isoform 1;
SEQ ID NO. 2: the amino acid sequence of the polypeptide that corresponds to the portion between amino acids 677 and 1098 of the human TCERG1 protein;
SEQ ID NO. 3: the amino acid sequence of the polypeptide that corresponds to the portion between amino acids 683 and 793 of the human TCERG1 protein;
SEQ ID NO. 4: the amino acid sequence of the polypeptide that corresponds to the portion between amino acids 751 and 770 of the human TCERG1 protein;
SEQ ID NO. 5: the amino acid sequence of the polypeptide that corresponds to the portion between amino acids 707 and 729 of the human TCERG1 protein;
SEQ ID NO. 6: the amino acid sequence of the polypeptide that corresponds to the portion between amino acids 730 and 750 of the human TCERG1 protein;

The proteins (polypeptides or peptides) can be used as antigens. Production of specific antibodies for the peptides identified makes it possible to carry out competitive assays in which the presence of antibodies specific to the antigen (auto-antibodies) in biological samples is detected by the capacity to sequester said antigen.

The proteins (polypeptides or peptides) can be produced in bacteria, in recombinant form obtained by techniques based on genetic engineering, or by chemical synthesis.

In cases in which chemical modifications or modifications by recombinant techniques (for example modifications such as acetylations, carboxylations, glycosylations, phosphorylations, amidations) do not alter the functionality of the proteins, are to be regarded as included in the present patent.

The marker identified and the respective consensus sequence, surprisingly, make it possible to distinguish between patients with MS and with other neurological diseases with a specificity of 90-98%. The sensitivity of 22-50% signifies that the antigen is potentially able to diagnose different states of the disease only to be found in certain patients.

Advantageously, another aspect of the present invention relates to a protein SEQ ID NO.2 as biological marker for monitoring the efficacy of therapeutic treatment in multiple sclerosis; and make it possible to monitor the parameters of the inflammatory state following treatment with interferon, and are correlated with the clinical state.

Until now, the main parameter of the efficacy of interferon treatment was reduction in the number of relapses. The advantage of the present invention is that it supplies biological markers for monitoring the efficacy of the treatment without having to wait to verify the relapses of the pathology.

The invention relates to a protein SEQ ID NO.2 as biological marker for distinguishing between patients with multiple sclerosis and patients with other neurological diseases such as polyneuropathies, polyneuritides, polyradiculoneuritides, encephalitides, myelitides, meningitides, leukoencephalopathies, vasculitides, Miller-Fisher and Guillain-Barré syndromes, amyotrophic lateral sclerosis, spastic tetraparesis, paraneoplastic neuropathies, Charcot-Marie-Tooth syndrome, spinal cord injuries, hydrocephalus, sub-arachnoid haemorrhages.

Surprisingly, in a preferred embodiment, the inventors of the present invention made it possible to improve the possibility of discriminating between patients with neurological symptoms of various kinds, common to various diseases affecting the nervous system, and patients with multiple sclerosis. Multiple sclerosis in fact displays a multiplicity of symptoms that can be confused with those of other neurological diseases, and the diagnostic techniques currently used do not allow this distinction to be made. Consequently there is a need to identify markers that make it possible in particular to distinguish between patients with neurological symptoms, rather than between multiple sclerosis patients and healthy individuals. According to another aspect of the invention, the protein SEQ ID NO.2 is a biological marker in protein platforms (arrays).

In a preferred embodiment, said protein platforms are miniaturized (protein chips). Miniaturization offers numerous advantages compared with the conventional techniques. These advantages comprise improvement in the accuracy and reproducibility of the data, shorter analysis times, minimum consumption of sample, potential for automation and integration of complex operational flows. Another aspect of the invention relates to the protein SEQ ID NO.2 in which said protein
is a biological marker for determining the individual immunological profiles.

These proteins can in fact, surprisingly, be used together with other polypeptides for constructing protein platforms (for example protein arrays) that are able to detect the presence of specific antibodies (auto-antibodies) and determine a patient's immune state and clinical profile.

In multiple sclerosis, it has been found to be very important to be able to discriminate between the various states of the disease. Another aspect of the invention relates to a protein SEQ ID NO.2, as biological marker for discriminating between different states of multiple sclerosis that are known to have different forms:
a. Relapsing-remitting form (RR);
b. Primary-progressive form (PP);
c. Secondary-progressive form (SP).

Another aspect of the invention relates to a method for the diagnosis of multiple sclerosis using the protein SEQ ID NO.2.

A preferred method for the diagnosis of multiple sclerosis using a protein SEQ ID NO.2 makes it possible to discriminate between patients with multiple sclerosis and patients with other inflammatory and non-inflammatory pathologies of the central nervous system or between patients before and after therapeutic treatment with interferon, which comprises the steps of:
a. providing a biological sample;
b. providing a protein SEQ ID NO.2 immobilized on a solid substrate;
c. bringing the sample of step a. into contact with the substrate of step b.;
d. detecting the reaction.

The peptides or the protein corresponding to the marker identified as TCERG1 can be used in all assays of the diagnostic type in which samples of biological origin (serum, liquor) can be analysed for their reactivity to said marker.

In particular, this relates to all assays in which the biological marker SEQ ID NO.2 can be used for identifying the presence of specific antibodies, i.e. capable of forming immune complexes with the marker, in biological samples. As an example, these antigen-antibody reactions can be detected with enzyme-linked immunosorbent assay (ELISA), radioimmunoassay (RIA), Western blot and LINE blot immunological assays, assays with protein microarrays.

Another aspect of the invention therefore relates to a method in which the diagnosis is performed by a technique selected from the group consisting of:
a. assays of the ELISA type;
b. assays of the radioimmunoassay (RIA) type;
c. assays of the Western blot and LINE blot type;
d. assays with protein microarrays.

Moreover, the protein sequences according to the present patent can be used for identifying the particular antigens present in biological samples by the production of antibodies specific to specified amino acid sequences. The immunological assays that can be used are similar to those enumerated above. Production of antibodies specific to the peptides identified makes it possible to carry out competitive assays in which the presence of antibodies specific to the antigen (auto-antibodies) in biological samples is detected by the capacity to sequester said antigen.

In a preferred method, control during execution of the assay takes place:
a. in the absence of antigen to detect the background of the reaction; and
b. in triplicate with a reference sample to be used as positive control, the value of which serves for standardizing the values of the biological samples under investigation.

The biological samples used in the present invention are preferably samples of serum or of cerebrospinal fluid.

A further aspect of the present invention relates to a diagnostic kit for the diagnosis of an autoimmune disease comprising a protein consisting of SEQ ID NO.2 that acts as antigen and a reagent for separate, simultaneous and successive use as a conjugated secondary antibody able to detect the antibodies present in the biological samples and which are reactive to the protein or antigen used.

An autoimmune disease preferably diagnosed by means of the diagnostic kit of the present invention is Multiple Sclerosis.

The diagnostic kit makes it possible, surprisingly, to discriminate the patient with multiple sclerosis or a specified autoimmune state. The kit should envisage, for example, supply of the antigen immobilized on a solid substrate; detection of reaction with specific antibodies present in biological samples (e.g. ELISA) should take place in parallel with two types of controls: execution of the assay (1) in the absence of antigen to detect the background of the reaction and (2) in triplicate with a reference sample to be used as positive control, the value of which serves for standardizing the values of the samples under investigation.

### EXAMPLES

### Example 1

### Biological samples

The serum samples and cerebrospinal fluid were obtained after obtaining informed consent, and were stored at -80°C. All the patients with multiple sclerosis had been diagnosed according to the McDonald criteria, and for the most part had the RR form (with relapses and remissions); the clinical and personal data are summarized in Table 1. The patients with other neurological diseases comprised: polyneuropathies, polyneuritides, polyradiculoneuritides, encephalitides, myelitides, meningitides, leukoencephalopathies, vasculitides, Miller-Fisher and Guillain-Barré syndromes, amyotrophic lateral sclerosis, spastic tetraparesis, paraneoplastic neuropathies, Charcot-Marie-Tooth syndrome, spinal cord injuries, hydrocephalus, sub-arachnoid haemorrhages.

The samples were kindly supplied by the Multiple Sclerosis Centre of Trieste and Cagliari and by the Neurology Clinic of Padua.

**Table 1**

| Clinical and personal data with respective mean values of the patients with multiple sclerosis recruited for selection of antigens and the following diagnostic validations. | | | | | | |
|---|---|---|---|---|---|---|
| **MS samples** | ***N*** | **Female/ male** | **Average age (range) in years** | **Diagnosis** | **Average duration (range) in years** | **EDSS range** |
| | | | | 20 RR | RR: 9.3 (3-28) | 0-3.5 (CIS and RR) 8 (PP |
| **Group 1** | 28 | 18/12 | 42.7 (21-66) | 7 CIS | CIS: 5 (4-7) | |
| | | | | 1 PP | PP: 27 | |
| **Group 2** | 90 | 71/19 | 40.4 (19-63) | RR | 12(1-36) | 0-6.5 |
| **Group 3** | 18 | 7/11 | 48.7 (35-67) | SP | 18.9 (6-35) | 3-7 |
| **Group 4** | 30 | 19/11 | 37.6 (22-54) | RR | 5.8 (1-20) | / |

### Bacterial strains

The bacterial strains used were as follows: *Escherichia coli* DH5αF' (Gibco BRL): F*'*/*endA1 hsd17* (r_{κ} ⁻m_{κ}⁺) *supE44 thi-1 recA1 gyrA* (Nal^{r}) *relA1Δ* (*lacZYA-argF*) *U169 deoR* (F80*dlacD-*(*lacZ)M15*)*,* for propagation of the phages and construction of the scFv (single-chain variable fragment) library; TOP10F': F'{*lac*lq*Tn*10(TetR)} *mcrA Δ*(*mrr-hsd*RMS*-mcr*BC) Φ80*lac*ZΔM15 Δ*lac*X74 *rec*A1 *ara*D139 Δ(*ara-leu*)7697 *ga*/U *gal*K *rps*L endA1 *nup*G*,* for constructing the cDNA library; BS1365: BS591 F' Kan [BS591: *recA1 endA1 gyrA96 thi*-1 *D lacU169 supE44 hsdR17 (lambda1 mm434 nin5 X 1-cre*)]*,* for recombination of the library.

### Example 2

### Production of a phage display library of scFv antibodies from cerebrospinal fluid of patients with MS

The next example describes obtaining a library of scFv antibodies by cloning of cDNA coding for variable regions of heavy (VH) and light (VL) antibody chains of two patients with multiple sclerosis.

The B cells were collected by centrifugation from the CSF of two RRMS patients; the total RNA was extracted and retrotranscribed into cDNA. The VH and VL regions were produced by PCR, assorted randomly and used for cloning in the pDAN5 vector as described by Sblattero et al. (2000). After transformation by electroporation into E. coli DH5αF', a library of 2x10⁴ independent clones was obtained, with a diversity of 30.8% and 72.7% respectively for the VH and VL chains.

### Methodology:

### Construction of the scFv library from CSF

The RNA for constructing the scFv library was obtained from B cells of cerebrospinal fluid of two RR patients. Both patients were positive for the presence of oligoclonal bands and were not undergoing treatment; one was a woman of 40 years (start of the disease at 36) with an equal EDSS; the other was a man of 29 years (start of the disease at 27) with an EDSS equal to 3.5.

The total RNA was extracted from a pellet of 2 × 10⁴ B cells using the system PicoPure™ RNA Isolation Kit (Arcturus). The first cDNA strand was synthesized using random hexamers and SuperScript TM III RT (Invitrogen), according to the supplier's instructions. Each family of VH and VL genes was amplified separately in a final reaction volume of 20 µL containing 1 U of ExTaq polymerase (TaKaRa) and the specific primer (Sblattero et al. 2000). In the case of the VH chains, the primer at 3' was specific to the IgGs. PCR was conducted for 30 cycles with the following conditions: denaturation for 30 s at 94°C, pairing for 30 s at 55°C, extension for 45 s at 72°C. The individual VH and VL genes were combined to obtain two equimolar VH and VL mixtures, which were amplified and assembled as described by Sblattero et al. (2000).

The assembled PCR products coding for scFV were then mixed with a phagemid vector pDAN5 digested with BssHII/Nhel enzymes at a molar ratio of 10:1 (fragments:vector) and submitted to ligation. The ligation mixture was transformed into DH5α F' E. coli cells by electroporation. Individual clones obtained by the transformation were analysed by PCR, enzymatic fingerprinting and sequencing.

### Example 3

### Construction of the vector by selection of ORF fragments

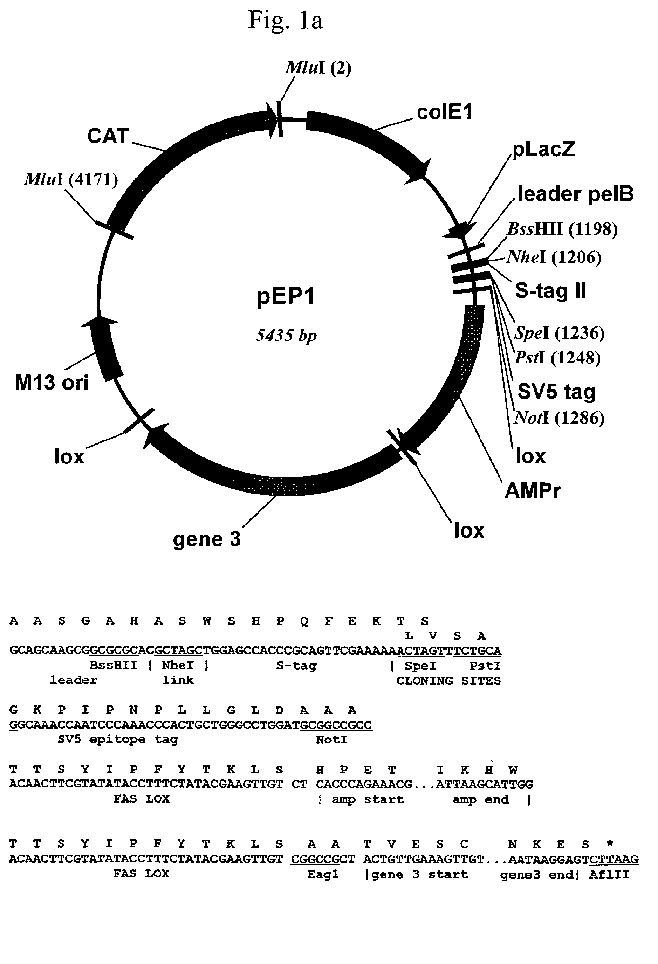

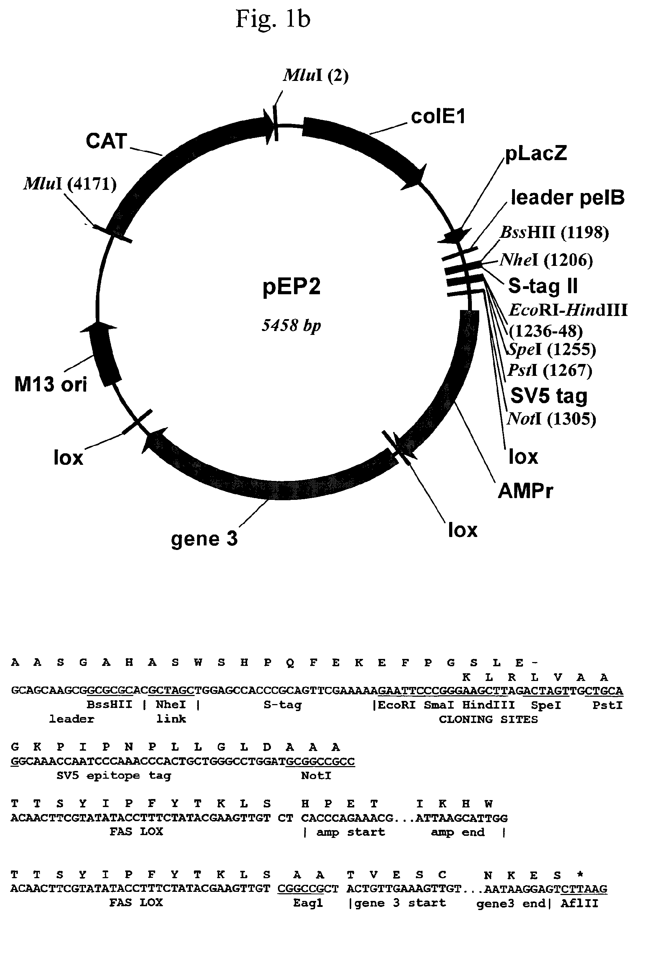

Starting from the skeleton of the vector pPAO2 (Zacchi et al., 2003), two new vectors were obtained, designated pEP1 and pEP2, by modifying the sequence of the polylinker. The essential characteristics of these vectors are shown in Fig. 1. The system for selecting ORF fragments by means of fusion with the β-lactamase gene was maintained.

The modifications introduced in the polylinker relate to: (a) the cloning strategy, as Spel sites, were inserted for pEP1, and EcoRI and HindIII, for pEP2, by restriction-mediated cloning and (b) the system for purification and detection of the coded polypeptides (the sequence Streptag II [IBA-Go] was positioned upstream of the cloning site, replacing the existing tag sequences). In particular, the pEP2 vector allows oriented cloning of cDNA, according to the system OrientExpress Random Primers Novagen, to increase the probability of cloning fragments with the correct reading frame from 1/18 to 1/9. Moreover, the vectors have two new restriction sites for direct subcloning of ORF inserts into expression vectors and the amber stop codon (TAG), present in pPAO2 between cDNA insert and β-lactamase, was removed to make selection of ORF fragments more efficient.

### Methodology

### Expression and purification of scFv antibodies

To produce recombinant scFv antibodies in soluble form, HB2151 cells were infected with phages of the antibody library from CSF and were grown at 37°C in 2xYT medium containing 100µg/mL of ampicillin up to an OD equal to 0.5. After adding IPTG to a final concentration of 0.5 mM, growth was continued overnight. After centrifugation at 4500 g for 20 min, the pellet was resuspended in 10 ml of lysis buffer (Tris-HCl 20 mM pH 8.0, NaCl 500 mM, imidazole 5 mM, Triton X100 0.1%) per gram of cells, together with lysozyme 100µg/mL and DNase 30 µg/ml, and incubated in ice for 60 min. Then the samples were centrifuged at 4500 g for 20 min to separate the included bodies from the soluble cellular proteins. The included bodies containing the scFvs were resuspended in 10 ml of solubilizing buffer (Tris-HCl 20 mM pH 8.0, NaCl 500 mM, imidazole 5 mM, TritonX100 0.1 % and urea 8M) and incubated for 1 hour at 4°C. The sample was centrifuged at 4500 g for 20 min and the scFvs were purified by affinity chromatography using NiNTA resin (IBA). Folding of the scFvs was carried out directly on the column using a linear gradient of urea from 8.0 M to 0 M. The renatured scFvs were eluted using a buffer containing Tris-HCl 20 mM pH 8.0, NaCl 500 mM and imidazole 300 mM.

### pEP vectors

The phagemid pEP1 is derived from pPAO2 (Zacchi et al. 2003) modified in order to contain a new polylinker sequence. The new polylinker was cloned into pPAO2, digested with BsshII and NotI, by insertion of two superposed oligonucleotides,
OLPE153
   (5'-CGCGCACGCTAGCTGGAGCCACCCGCAGTTCGAAAAAACTAGTTTC TG
      CAGGCA-3')
   and
OLPE135
   (5'-GGCCGCATCCAGGCCCAGCAGTGGGTTTGGGATTGGTTTGCCTGCAGA AACTAG-3').

In particular, the sequence for StreptagII was introduced, and the restriction sites for EcoRI and Spel.

The phagemid pEP2 was obtained from pEP1 in order to permit directed cloning.

A new polylinker sequence was inserted using the oligonucleotides
Poly1
   (5'-AGCTCGGGTCTCGAGCTAGCCAAATTCTATTTCAAGGAG-3')
   and
Poly2
   (5'-CCGGGCTGCAGCAACTAGTCTAAGCTTCCCGGGAATTCTTTTTCGA ACT
      GCGGGTGGCTC-3').

### Example 4

### Construction of a phage display library enriched in ORF cDNA fragments from human brain

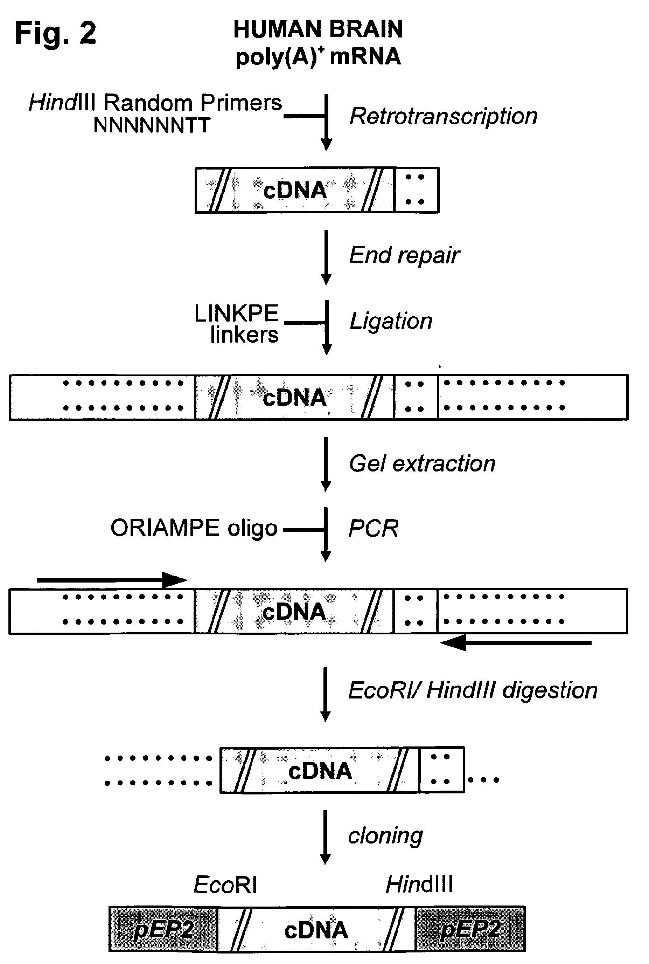

The scheme for constructing the phage display library from human brain is shown in Fig. 2. The first oriented strand of cDNA was synthesized starting from mRNA poly(A)+ of human brain obtained from BD Bioscience, in particular of a total of eight forebrains of healthy Caucasian males who died unexpectedly. The system for directed cloning of cDNA consists of using, during retrotranscription, oligonucleotides with a random sequence, but with a conserved sequence (TT) at 5' (OrientExpress Random Primers Novagen). With an mRNA/oligonucleotide ratio of 1/2.5, cDNA fragments were obtained from 100 bp to 2 kb. Both the first and the second cDNA strand were synthesized in the presence of 5-methyl-dCTP to protect the internal restriction sites EcoRI and HindIII from subsequent digestions. Next, after ligation with specific linkers "EcoRI/HindIII" designed by us, for which a Hindlll site is recreated only at the 3' end owing to the two A's inserted in the synthesis of the first strand, the oriented cDNAs of length between 300 and 800 bp were purified with agarose gel, amplified, submitted to digestion with EcoRI and HindIII and cloned into pEP2. The ligation mixture was transformed into electrocompetent TOP10 F' E. coli cells, then selected for the presence of ORF inserts by plating in the presence of 12 µg/ml of ampicillin. 1.35x10⁵ independent clones were obtained and the library was recombined to remove the β-lactamase gene (Zacchi et al., 2003). The average length, diversity and identity of the clones of the library were evaluated by PCR, restriction fingerprinting and random sequencing.

### Methodology

### Construction of the phage display library of human brain cDNA

The cDNA library was constructed from 1 µg of human brain poly(A)+ RNA (Clontech, cod. 6516-1). Synthesis of the first cDNA strand used 2.5 µg of Random Primers HindIII (Novagen), 200 U of SuperScriptIII RT (Invitrogen) and 200 U of RNaseOUT (Invitrogen), in the presence of methylated dNTPs 0.5 mM, according to the manufacturer's instructions. The second strand was synthesized with 23 U of DNA polymerase I (Promega) and 0.8 U of RNase H (USB). The reaction was incubated at 14°C for 2 hours and purified with phenol:chloroform. After end repair by adding 1.5 U of T4 DNA polymerase (NEB), in the presence of 0.4 mM dNTP and incubation at 12°C for 20 min, the cDNA was ligated to the linkers

### LINKPE

(LINKPE53pcr 5'-AGGGGAGGGGGCTTGAATTCAAGC-3' and
LINKPE35pcr 5'-CTCCCCT pGCTTGAATTCAAGCCCC-3'), pre-incubated at 95°C for 2 min, 65°C for 5 min and 42°C for 10 min. The reaction was carried out at 16°C overnight. The cDNA fragments in the range 300-800 bp were extracted and purified with agarose gel and amplified by the primers complementary to the linker sequence
ORIAMPEFOR (5'-GAGGGGGCTTGAATTCAAGC-3') and
ORIAMPEREV (5'-GGGGGCTTGAATTCAAGCTT-3').

.PCR was carried out in the presence of 1 U of DNA polymerase Phusion Hot Start (Finnzymes) for 30 cycles in the following conditions: denaturation for 10 s at 98°C, pairing for 30 s at 60°C, extension for 45 s at 72°C. After purification, the cDNA fragments were digested, separately, with 30 U of Hindlll (Promega) and with 30 U of EcoRI (NEB). Both reactions were incubated at 37°C for 3 hours. The fragments were then mixed with the pEP2 vector, cut both with HindIII and EcoRI, in a molar ratio equal to 10:1 and ligated using T4 DNA ligase (NEB). The ligation mixture was purified with phenol:chloroform and divided into 8 aliquots, each of which was used for transforming 40 µL of electrocompetent E. coli TOP10F' cells (Invitrogen). The transformation mixture was plated on medium containing 25 µg/ml of cloramfenicol and 12 µg/ml of ampicillin. The resultant colonies were analysed by PCR, enzymatic fingerprinting and sequencing.

After selection on ampicillin plates, the β-lactamase gene was removed by infecting BS1365 cells (which express Cre-recombinase constitutively) with the phages of the library in 10 ml of 2xTY, 25 µg/ml of kanamycin, 1% of glucose at 37°C up to growth equal to OD of 0.5. After adding 25 µg/ml cloramfenicol, recombination took place during bacterial growth at 30°C overnight. The next day, the bacteria were diluted 1/20 and were grown to OD of 0.5 at 37°C. After infection with the helper phage M13K07 at 37°C for 30 min and growth at 30°C overnight, the phages produced were used for infecting cells of E.coli DH5αF'. The clones obtained represent the library of selected ORF fragments.

### Example 5

### Selection of the human brain cDNA library with the scFv library from CSF of patients with MS

Using the phage library of scFv from CSF of patients as coating of an immunotube, three selection cycles were performed, obtaining a 160-fold enrichment of specific clones.

From the last selection cycle, 94 clones selected at random were analysed by ELISA to evaluate their reactivity in comparison with the total soluble scFvs. 19 clones with an optical density (OD) above 0.2 were analysed by PCR; 17 of these had a correct insert and were sequenced. Sequence analysis revealed that all the clones were correctly "in-frame". Some clones corresponded to superposed sequences of the same protein; the complete list of proteins identified as candidate autoantigens of MS are shown in Table 2; some of these constitute mimotopes. In particular, identification of the protein DDX24 will be revealed, as this same protein was identified in two other selections of the same human brain antigen library performed, respectively, with a set of sera and a set of CSF of RR patients.

**Table 2:**

| List of antigens identified in the procedure for selection of the antigen phage library with the antibody library from CSF of patients with multiple sclerosis; the table shows the identifying acronyms of the clones, the access number of the respective nucleotide sequence in the NCBI database, description of the sequence coding for the antigen fragment of the clone, in the case of homology at nucleotide level (cds) or of the protein homologous to the peptide encoded by the sequence identified (mimotope), and the corresponding amino acids. | | | | |
|---|---|---|---|---|
| ***Clones*** | ***NCBI access number*** | ***Description*** | ***CDS*** | ***a.a.*** |
| HBscFvA1 | NM 020414.3 | Homo sapiens DEAD (Asp-Glu-Ala-Asp) box polypeptide 24 (DDX24) | real | 87-215 |
| HBscFvB10 | | | | |
| HBscFvE3 | | | | 99-215 |
| HBscFvA5 | | | | |
| HBscFvB5 | | | | 140-229 |
| HBscFvH5 | | | | |
| HBscFvB7 | NM 024863.4 | Homo sapiens transcription elongation factor A (SII)-like 4 (TCEAL4), transcript variant 1 | real | 98-148 |
| HBscFvE5 | NM 006706.3 | Homo sapiens transcription elongation regulator 1 (TCERG1), transcript variant 1 | real | 683-793 |
| HBscFvE8 | NM 174889.2 | Homo sapiens NDUFA12-like (NDUFA12L) | real | 1-118 |
| HBscFvG2 | NM 002128.3 | Homo sapiens high-mobility group box 1 (HMGB1) | real | 7-95 |
| HBscFvG6 | gbAAC51279.1 | Homo sapiens Putative p150 (RT like) | mimotope | 481-500 |
| HBscFvG7 | qbABX35540.1 | DEAD/DEAH box helicase domain protein (Delftia acidovorans) | mimotope | 452-531 |
| HBscFvC12 | dbjBAD93163.1 | caveolin 1 variant [Homo sapiens] | mimotope | 30-45 |
| HBscFvA8 | | | | |
| HBscFvH11 | GENE ID: 2319FLOT2 | Flotillin-2 [Homo sapiens] | mimotope | 28-38 |
| HBscFvB6 | | | | |
| HBscFvF4 | refXP 729762.1 | Senescence-associated protein [Plasmodium yoelii] | mimotope | 144-158 |

### Methodology

### Selections between cDNA and scFv libraries

For each cycle, a well of a microtitre plate (Costar) was coated with 9x10¹² phages of the scFv library in carbonate buffer (NaHCO₃ 1M pH 9) at 4°C overnight. After washing with PBS, saturation was carried out for 1 hour at room temperature with BSA 3% in PBS.

The phages of the cDNA library, purified by means of PEG, were diluted in an equal volume of BSA 6% in PBS, incubated for 30 minutes at room temperature, added to the well and incubated for 30 min with stirring and a further 90 min, still at room temperature.

For the first cycle, 10 washings were performed with PBS-Tween (PBST) 0.5% and 10 with PBS. The phages were eluted with glycine 200 mM pH 2.2 in BSA 2%, neutralized with Tris-HCl 1 M pH 9 and used for infecting E. coli DH5αF' cells, grown to OD of 0.5, for 40 min at 37°C. The phages were recovered by infection with the helper phage M13K07, in 10 ml of 2xYT with 25 µg/ml cloramfenicol and 25 µg/ml kanamycin, and incubation at 30°C overnight.

The procedure was similar for the other cycles, except that for the second cycle, 10 washings were performed with PBST 0.1 % and 10 with PBS, while for the third cycle there were 20 washings with PBST 0.1% and 20 with PBS. For monitoring the enrichment of specific clones, the amount of phages selected at input and output for each cycle was titrated and the input/output ratio was determined. After selection, 95 individual clones selected at random were submitted to phage-ELISA analysis.

### Example 6

### The protein TCERG1 as putative autoantigen of MS

Starting from the protein TCERG1 (SEQ ID NO.1), the sequence according to the present invention comprises a portion of protein TCERG1 (otherwise known as CA150 or TAF2S) corresponding to the amino acids from position 677 to 1098 (SEQ ID NO.2) and respective possible modifications as deducible from the annotations present in public databases, available from the sites given below and summarized hereunder, and from the definition of a consensus sequence given above.

Access codes of the protein:
HGNC:15630
Ensembl:ENSG00000113649
HPRD:10393

### Reference sites

http://www.ncbi.nlm.nih.gov/nuccore?Db=gene&Cmd=retrieve&dopt=full report&lis t uids=10915&log$=databasead&logdbfrom=nuccore
http://www.genenames.org/data/hgnc data.php?hgnc id=15630
http://www.hprd.org/summary?hprd id=10393&isoform id=10393 1&isoform nam e=Isoform 1

### Alternative names

- transcription elongation regulator 1
- TATA box binding protein (TBP)-associated factor, RNA polymerase II, S, 150kD
- TATA box binding protein associated factor 2S
- Transcription factor CA150
- co-activator of 150 kDa

Complete sequence corresponding to the two isoforms 1 and 2 (access code: NP_006697 and NP_001035095)

Single nucleotide polymorphisms (SNPs) present in the region
- rs12186370: SNP synonym (G/A), corresponding to the third base of the codon coding for the a.a. Thr [T] in position 976;
- rs4705103: SNP synonym (G/A), corresponding to the third base of the codon coding for the a.a. Ser [S] in position 1040;

### Consensus sequence between two antigens

The following consensus sequence was constructed taking into account the homology of the antigen TCERG1 isolated SEQ ID NO:3, included in the sequence of the human TCERG1 protein and corresponding to the a.a. 683-793, with another antigen, DDX24, identified in the selection described and characterized by a high reactivity to biological samples from patients with MS. The alternative amino acids are indicated in the column.

Fig. 3 shows the portions of maximum homology between the two proteins TCERG1 and DDX24 (from 57 to 67%); bearing in mind that these two antigens were found to be more frequent in the selections of the phage library with the patients' antibodies, these portions might correspond to epitopes that are more recognized, around which peptides could be synthesized, to be used in diagnostic tests.

The consensus sequence of the invention of polypeptide SEQ ID NO.2 takes account of the regions of homology with another antigen identified, as illustrated above, and is shown below.

### Example 7

### Production of the recombinant protein

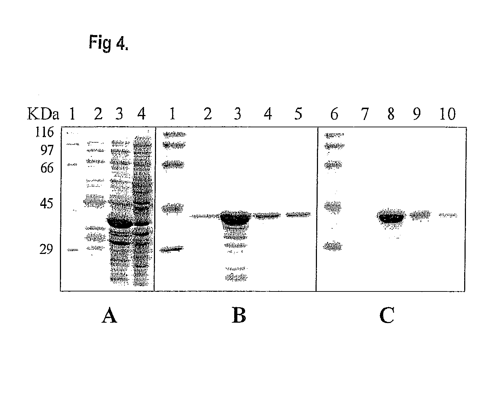

The cDNA coding for a portion of the human protein TCERG1, corresponding to the amino acids 677-1098 and comprising the epitope identified (a.a. 683-793), was cloned in the pASK45 expression vector (IBA). The cDNA was obtained by PCR from human brain cDNA using oligonucleotides designed on the sequence of the corresponding mRNA. Expression took place in cells of E.coli Rosetta 2, after optimization of the induction times. The protein was purified by affinity chromatography in two successive passes to obtain the complete form (with two purification tags at the ends) and at higher degree of purity (Fig. 4).

### Production of recombinant antigens

The cDNA of TCERG was obtained by PCR from human brain total cDNA using specific primer designed on the basis of the sequences available in the database: TCERG For
(5'-AAAATTCAGCTTTGATTTCAACGTGGGAGAAG-3')
and TCERG Back
(5'-TTCCTGCAGCCTTTTGTTGATGTGCTCCGTGG-3').

The cDNA was purified from gel, digested successively with EcoRI and Pstl (NEB), ligated to the vector pASK-45plus (IBA) and transformed into Rosetta2 cells.

The recombinant protein rTCERG (677-1098) was induced by a clone containing the vector TCERG (677-1098)-pASK45plus with anhydrotetracycline 200 ng/ml for 3 hours at 37°C. The bacterial pellet was resuspended and the protein was purified as described in the manufacturer's protocol for purification by Strep-tagII (IBA). The protein was further purified using the second tag at the C-terminal. The sample was diluted 1:10 in solution A (20 mM Tris-HCl pH 8, 50 mM NaCl, 5 mM imidazole) and loaded on a column NiNTA (Amersham) equilibrated with the same buffer. After washing with 15 ml of solution B (20 mM Tris-HCl pH 8, 50 mM NaCl, 0.1 % Triton X-100 (v/v), 20 mM imidazole) and 10 ml of solution A, the sample was eluted with 10 ml of elution buffer (20 mM Tris-HCl pH 8, 50 mM NaCl, 300 mM imidazole). All the aliquots eluted were checked by electrophoresis.

### Example 8

### Synthesis of peptides

Three peptides of 21-23 amino acids, corresponding to three different, superposed portions (Fig. 3) of the TCERG1 antigen identified (peptide TCERG1-A: a.a. 707-729 (SEQ ID NO.5); peptide TCERG1-B: a.a. 730-750 (SEQ ID NO.6); peptide TCERG1-C: a.a. 751-770) (SEQ ID NO.4), were synthesized (Gen Way Biotech, Inc), adopting the following criteria: net charge different from 0, percentage of acidic or basic amino acids greater than 25% and of hydrophobic amino acids less than 25%, absence of cysteines (to avoid formation of any disulphide bridges).

### Example 9

### Primary phage-ELISA

Selected single clones were cultured in a titration plate with 96 round-bottomed wells up to OD of 0.5. Each clone was infected with a helper phage M13K07 at 37°C for 30 min and was incubated at 30°C overnight to allow production of the phages. In parallel, 96 flat-bottomed wells of a titration plate (Costar) were covered by incubation overnight at 4°C with the soluble form of the scFvs of the antibody library from patients' cerebrospinal fluid and were saturated with BSA 2% in PBS for 1 hour at room temperature. Then each supernatant of the phage cultures was diluted 1:1 (v/v) in BSA 4%-PBS and incubated for 90 min at room temperature. After 3 washings with PBST 0.1% and 3 with PBS, an anti-M13 monoclonal antibody, conjugated with peroxidase (Amersham Pharmacia Biotech), diluted 1:3000 in BSA 2%-PBS, was added and incubated for 1 hour at room temperature. After 3 washings with PBST 0.1% and 3 with PBS, a colorimetric reaction was started with 3,3',5,5'-tetramethylbenzidine (TMB, Sigma) and the plates were read at 450 nm.

Phage helper M13K07 was used as internal negative control. The immunoreactivity was measured for each phage clone as the OD ratio between the sample and the helper phage.

### Analysis of the validity of TCERG1 as biomarker of MS

For confirming the recognition and specificity of the TCERG1 antigen, secondary ELISA assays were performed using single samples of serum or CSF of patients with MS and other neurological diseases (OND) as well as from healthy donors. The following assays (Table 3) were performed with the antigen according to the present invention in three different formats:
a) protein fragment corresponding to amino acids 683-793 of human TCERG1 (peptide TCERG1-A: SEQ ID NO.5) exposed on the surface of a phage M13 (assay 1);
b) recombinant protein corresponding to amino acids 677-1098 of human TCERG1 (peptide TCERG1-B: SEQ ID NO.6) (assay 2);
c) peptide corresponding to amino acids 751-770 (peptide TCERG1-C SEQ ID NO.4) of human TCERG1 (assays 3 and 4): this peptide showed greater reactivity to serum samples from patients with MS in preliminary tests in which all three peptides TCERG1-A, B and C were assayed individually.

(1) "phage ELISA" on 17 CSF samples from patients with MS and 17 with OND: the two groups were found to be statistically different (p = 0.0009); ROC analysis for assessing the diagnostic value of TCERG1 gave a likelihood ratio (LR) above 8, corresponding to a specificity of 94% and a sensitivity of 47% for a cut-off value equal to 2.12. Subjects who were true positives (PPV) and true negatives (NPV) were, respectively, 89% and 64% (Fig. 5).
(2) ELISA on 20 serum samples from patients with MS and 20 with OND D: the two groups were found to be statistically different (p < 0.0001); ROC analysis for assessing the diagnostic value of TCERG1 gave a likelihood ratio (LR) above 11, corresponding to a specificity of 95% and a sensitivity of 55% for a cut-off value equal to 1.45. Subjects who were true positives (PPV) and true negatives (NPV) were, respectively, 92% and 68% (Fig. 6).
(3) ELISA on 41 serum samples from patients with MS and 40 with OND: the two groups were found to be statistically different (p = 0.01); ROC analysis for assessing the diagnostic value of TCERG1 gave a likelihood ratio (LR) above 2.22, corresponding to a specificity of 90% and a sensitivity of 22% for a cut-off value equal to 1.45. Subjects who were true positives (PPV) and true negatives (NPV) were, respectively, 69% and 53% (Fig. 7).
(4) ELISA on 30 serum samples from patients with MS, 40 with OND and 10 healthy subjects: the three groups were found to be statistically different (p = 0.01); ROC analysis for assessing the diagnostic value of TCERG1 gave a likelihood ratio (LR) above 12, corresponding to a specificity of 98% and a sensitivity of 30% for a cut-off value equal to 1.44. Subjects who were true positives (PPV) and true negatives (NPV) were, respectively, 90% and 65% (Fig. 8).

**Table 3**

| Synopsis of the statistical data for the various secondary ELISA assays carried out using single samples of serum or CSF of patients with multiple sclerosis (MS), other neurological diseases (OND) and from healthy donors (HD). The cut-off represents the threshold value of the optical density ratio, obtained for each sample in the presence and absence of antigen, used for calculating the values of specificity, sensitivity, PPV, NPV and LR+. | | | | | | | | | | | | | |
|---|---|---|---|---|---|---|---|---|---|---|---|---|---|
| Assay | *Antigen* | *Samples* | *MS* | *Ctrl* | *HD* | *cut-off* | *Specificity* | *Sensitivity* | *PPV* | *NPV* | *LR+* | *Significance* (*P*) | *Area ROC* |
| 1 | phage | CSF | 17 | 17 | 0 | 2,12 | 94% | 47% | 89% | 64% | 8 | 0,0009 | 0,8 |
| 2 | prot | sera | 20 | 20 | 0 | *1,45* | 95% | 55% | 92% | 68% | 11 | <0.0001 | 0,93 |
| 3 | peptide | sera | 41 | 40 | 0 | *1,45* | *90%* | 22% | 69% | 53% | 2,2 | 0,01 | 0,72 |
| 4 | peptide | sera | 30 | 40 | 10 | 1,44 | 98% | *30%* | 90% | 65% | 12 | 0,01 | 0,71 |

### Secondary Phage-ELISA

The wells of a titration plate with 96 flat-bottomed wells (Costar) were covered by incubation overnight at 4°C with the antibodies present in the serum (diluted 1:100 in PBS) and in the CSF (diluted 1:5 in PBS) of individual patients or healthy donors. After saturation with BSA 2% in PBS for 1 hour at room temperature, 10⁹ or 10¹⁰ phages, precipitated with PEG, of a single clone selected in the human brain library, diluted in BSA 2% in PBS, were added and incubated for 1 hour at room temperature. The protocol was continued as in primary Phage-ELISA.

For each sample, a helper phage M13K07 was used as negative control and the immunoreactivity was measured as the OD ratio between the phage tested and the helper phage.

### Example 10

### Test of specificity of the TCERG1 antigen

In order to confirm the specific recognition of the peptide TCERG1-C by the sera from MS, a competitive ELISA assay was performed. One of the MS sera that proved statistically "true positive" and one of the OND sera statistically "true negative" were pre-incubated with increasing concentrations of peptide TCERG1-C (from 0 to 82.5 µM). After incubation for 30 minutes, the reactivity of the serum was evaluated by ELISA using the same peptide. Effective, progressive competition was observed in the case of pre-incubation of the MS serum with the peptide, whereas this is absent in the case of the OND serum (Fig. 9). In fact, in the case of the MS serum, at the higher competitor concentration, the value of OD is similar to that of the control sample, which remains almost constant, demonstrating a specificity of recognition of the peptide TCERG1-C by the MS serum.

### ELISA with the recombinant antigen

Using the recombinant protein as antigen, ELISA was performed by coating each well of the microtitre plate with 1 µg of the protein produced by incubation overnight at 4°C. An equivalent number of wells were incubated with only the buffer used for resuspending the proteins, as negative control of the reaction. After saturation with BSA 2% in PBS for 1 hour at room temperature, a different biological sample was added to each well (serum diluted 1:100 or CSF diluted 1:5 in BSA 2% in PBS) and was left for 1 hour at room temperature. After 3 washings with PBST 0.1% and 3 with PBS, an anti-human-IgG antibody, conjugated with peroxidase (Dako), diluted 1:1000 in BSA 2%-PBS, was added and incubated for 1 hour at room temperature. After 3 washings with PBST 0.1% and 3 with PBS, a colorimetric reaction was started with 3,3',5,5'-tetramethylbenzidine (TMB, Sigma) and the plates were read at 450 nm.

The immunoreactivity of each sample was evaluated as the ratio of OD detected with the antigen present or absent.

### ELISA with peptides

The ELISA performed using single peptides as antigen is identical to that in which whole recombinant protein is used, except that special plates were used, functionalized for attachment of the peptides: Reacti-Bind™ plates (Pierce). Each well was coated with 2 µg of peptide, whereas no peptide was added in the negative controls. The protocol is similar to that described above.

In competitive ELISA, the biological samples to be tested were a serum of a patient with MS and that of a patient with another neurological disease; both were pre-incubated with increasing concentrations of the same peptide used for coating the wells (0 M; 0.0825 M; 0.825 M; 8.25 M; 82.5 M), for 30 min at room temperature. Next, the reactivity of the peptide was determined as described above.

### Statistical analysis

Statistical analysis was performed using the software GraphPad Prism version 4.0. The significance of the differences between the groups of patients was evaluated using the t-test for unpaired data (one-sided), in the case of normal distributions, or the Mann-Whitney test (one-sided), if normal distribution is absent for at least one of the two groups; in the case of three groups, an ANOVA analysis was carried out. A value of p <0.05 was regarded as statistically significant. ROC analysis was carried out for determining the threshold values, of sensitivity and specificity, and of LR+.

### REFERENCES

Berger, Rubner, Schautzer, Egg, Ulmer, Mayringer, Dilitz, Deisenhammer, Reindl. Antimyelin antibodies as a predictor of clinically definite multiple sclerosis after a first demyelinating event. N Engl J Med. 349:139-145 (2003).
Khalil, Reindl, Lutterotti, Kuenz, Ehling, Gneiss, Lackner, Deisenhammer, Berger. Epitope specificity of serum antibodies directed against the extracellular domain of myelin oligodendrocyte glycoprotein: Influence of relapses and immunomodulatory treatments. J. Neuroimmunol. 174: 147-156 (2006).
Klawiter, Cross. B cells: no longer the nondominant arm of multiple sclerosis. Curr. Neurol. Neurosci. Rep. 7: 231-238 (2007).
Kuhle, Pohl, Mehling, Edan, Freedman, Hartung, Polman, Miller, Montalban, Barkhof, Bauer, Dahms, Lindberg, Kappos, Sandbrink. Lack of association between antimyelin antibodies and progression to multiple sclerosis. N Engl J Med. 356:371-8. (2007).
Lalive, Menge, Delarasse, Della Gaspera, Pham-Dinh, Villoslada, von Budingen, Genain. Antibodies to native myelin oligodendrocyte glycoprotein are serologic markers of early inflammation in multiple sclerosis. Proc. Natl. Acad. Sci. USA. 103: 2280-2285 (2006).
Lim, Berger, Reindl, Dalton, Fernando, Keir, Thompson, Miller, Giovannoni. Anti-myelin antibodies do not allow earlier diagnosis of multiple sclerosis. Mult Scler. 11:492-4. (2005).
Magliozzi, Howell, Vora, Serafini, Nicholas, Puopolo, Reynolds, Aloisi. Meningeal B-cell follicles in secondary progressive multiple sclerosis associate with early onset of disease and severe cortical pathology. Brain. 130: 1089-1104 (2007). McMahon, Bailey, Castenada, Waldner, Miller. Epitope spreading initiates in the CNS in two mouse models of multiple sclerosis. Nat. Med. 11: 335-339 (2005). Polman, Killestein. Anti-myelin antibodies in multiple sclerosis: clinically useful? J. Neurol. Neurosurg. Psychiatry. 77: 712 (2006).
Ratchford, Calabresi. The diagnosis of MS: white spots and red flags. Neurology. 70: 1071-1072 (2008).
Reindl, Khalil, Berger. Antibodies as biological markers for pathophysiological processes in MS. J. Neuroimmunol. 180: 50-62 (2006).
Salzer J, Svenningsson A, Sundström P. Neurofilament light as a prognostic marker in multiple sclerosis. Mult Scler. 16: 287-292 (2010).
Sblattero, Bradbury. Exploiting recombination in single bacteria to make large phage antibody libraries. Nat. Biotechnol. 18: 75-80 (2000).
Svejgaard. The immunogenetics of multiple sclerosis. Immunogenetics. 60: 275-286 (2008).
Zacchi, Sblattero, Florian, Marzari, Bradbury. Selecting open reading frames from DNA. Genome Res. 13, 980-990 (2003).
Zhou, Srivastava, Nessler, Grummel, Sommer, Bruck, Hartung, Stadelmann, Hemmer. Identification of a pathogenic antibody response to native myelin oligodendrocyte glycoprotein in multiple sclerosis. Proc. Natl. Acad. Sci. USA. 103: 19057-19062 (2006).

## Claims

1. A protein consisting of SEQ ID NO.2 for use as a biological marker for the diagnosis of multiple sclerosis.

2. The protein according to claim 1, wherein said protein is a biological marker for monitoring the efficacy of the therapeutic treatment of multiple sclerosis.

3. The protein according to claim 1, wherein said protein is a biological marker for monitoring inflammatory state parameters, after by interferon treatment.

4. The protein according to claim 1, wherein said protein is a biological marker for distinguishing patients with multiple sclerosis from patients with other neurological diseases such as polyneuropathies, polyneuritis, polyradiculoneuritis, encephalitis, myelitis, meningitis, leukoencephalopathies, vasculitis, Miller Fisher's Guillain Barrè's syndromes, amyotrophic lateral sclerosis, spastic tetraparesis, paraneoplastic neuropathies, Charcot Marie Tooth's syndrome, spinal cord injuries, hydrocephalus, subaracnoidea hemorrhages.

5. The protein according to claim 1, wherein said protein is a biological marker in protein platforms (array).

6. The protein according to claim 5, wherein said protein platforms are protein chips.

7. The protein according to claim 1, wherein said protein is a biological marker for determining the individual immunological profiles.

8. The protein according to claim 1, wherein said protein is a biological marker for discriminating between different states of multiple sclerosis; preferably wherein said states are selected for the group consisting of:
a. relapsing-remitting form (RR);
b. primary form; and
c. secondary-progressive form.

9. A method for diagnosing multiple sclerosis by using a protein consisting of SEQ ID NO:2.

10. The method according to claim 9, comprising the steps of:
a. providing the protein consisting of SEQ ID NO:2 immobilized onto a solid substrate;
b. contacting a biological sample previously obtained with the substrate from step a.
c. detecting the reaction.

11. A method according to claim 10, wherein said step c. is carried out by means of a technique selected from the group consisting of:
a. ELISA-type assays;
b. radioimmunological-type (RIA) assays;
c. immunological-type (Western Blot and LINE blot) assays;
d. protein microarrays assays.

12. A method according to any one of claims from 9 - 11, for discriminating between patients with neurological symptoms and patients with multiple sclerosis.

13. A method according to claim 10, wherein said biological sample is a serum or liquor sample.

14. Diagnostic kit comprising a protein consisting of SEQ ID NO:2, which serves as an antigen and a reagent for separate, simultaneous and consecutive use, for diagnosing multiple sclerosis

## Patentansprüche

1. Protein, bestehend aus SEQ ID NO: 2 zur Verwendung als biologischer Marker zur Diagnose von multipler Sklerose.

2. Protein gemäß Anspruch 1, wobei genanntes Protein ein biologischer Marker zur Überwachung der Wirksamkeit der therapeutischen Behandlung von multipler Sklerose ist.

3. Protein gemäß Anspruch 1, wobei genanntes Protein ein biologischer Marker zur Überwachung der Parameter der entzündlichen Zustände nach Interferon-Behandlung ist.

4. Protein gemäß Anspruch 1, wobei genanntes Protein ein biologischer Marker ist, um Patienten mit multipler Sklerose von Patienten mit anderen neurologischen Erkrankungen wie etwa Polyneuropathien, Polyneuritis, Polyradikuloneuritis, Enzephalitis, Myelitis, Meningitis Leukoenzephalopathien, Vaskulitis, Miller-Fisher-Guillain-Barre-Syndrome, amyotropher Lateralsklerose, spastischer Tetraparese, paraneoplastischen Neuropathien, Morbus Cahrcot-Marie-Tooth, Rückenmarksverletzungen, Hydrozephalus oder subarachnoidalen Blutungen zu unterscheiden.

5. Protein gemäß Anspruch 1, wobei genanntes Protein ein biologischer Marker in Proteinplattformen (Array) ist.

6. Protein gemäß Anspruch 5, wobei genannte Proteinplattformen Proteinchips sind.

7. Protein gemäß Anspruch 1, wobei genanntes Protein ein biologischer Marker zur Bestimmung von individuellen, immunologischen Profilen ist.

8. Protein gemäß Anspruch 1, wobei genanntes Protein ein biologischer Marker zur Unterscheidung verschiedener Verlaufsformen der multiplen Sklerose ist, vorzugsweise wobei genannte Verlaufsformen ausgewählt sind aus der Gruppe bestehend aus:
a. schubförmig remittierende Form (RR);
b. primäre Form und
c. sekundär-progrediente Form.

9. Verfahren zur Diagnose von multipler Sklerose unter Verwendung eines Proteins bestehend aus SEQ ID NO: 2.

10. Verfahren gemäß Anspruch 9, umfassend die folgenden Schritte:
a. Bereitstellen des Proteins, bestehend aus SEQ ID NO: 2, das auf einem festen Substrat immobilisiert ist,
b. In-Kontakt-Bringen einer vorher erhaltenen, biologischen Probe mit dem Substrat aus Schritt a,
c. Nachweisen der Reaktion.

11. Verfahren gemäß Anspruch 10, wobei genannter Schritt c mittels einer Technik durchgeführt wird, die ausgewählt ist aus der Gruppe bestehend aus:
a. ELISA-artigen Assays,
b. radioimmunologisch-artigen (RIA) Assays,
c. immunologisch-artigen Assays (Western Blot und LINE Blot),
d. Protein-Mikroarray-Assays.

12. Verfahren gemäß einem der Ansprüche von 9 bis 11 zur Unterscheidung von Patienten mit neurologischen Symptomen und Patienten mit multipler Sklerose.

13. Verfahren gemäß Anspruch 10, wobei genannte biologische Probe ein Serum oder eine Körperflüssigkeitsprobe ist.

14. Diagnostisches Kit, umfassend ein Protein, bestehend aus SEQ ID NO: 2, das als ein Antigen dient, und ein Reagenz zur separaten, gleichzeitigen und aufeinanderfolgenden Verwendung zur Diagnose von multipler Sklerose.

## Revendications

1. Protéine consistant en SEQ ID NO:2, pour une utilisation en tant que marqueur biologique pour le diagnostic de la sclérose en plaques.

2. Protéine selon la revendication 1, dans lequel ladite protéine est un marqueur biologique pour surveiller l'efficacité du traitement thérapeutique de la sclérose en plaques.

3. Protéine selon la revendication 1, dans lequel ladite protéine est un marqueur biologique pour la surveillance des paramètres de l'état inflammatoire, à la suite d'un traitement par interféron.

4. Protéine selon la revendication 1, dans lequel ladite protéine est un marqueur biologique pour distinguer les patients atteints de sclérose en plaques des patients atteints d'autres maladies neurologiques telles que les polyneuropathies, la polynévrite, la polyradiculonévrite, l'encéphalite, la myélite, la méningite, les leucoencéphalopathies, la vascularite, les syndromes de Guillain Barre et de Miller Fisher, la sclérose latérale amyotrophique, la tétraparésie spastique, les neuropathies paranéoplasiques, le syndrome de Charcot-Marie-Tooth, les blessures de la moelle épinière, l'hydrocéphalie, les hémorragies sous-arachnoïdiennes.

5. Protéine selon la revendication 1, dans lequel ladite protéine est un marqueur biologique dans les plates-formes de protéines (réseau).

6. Protéine selon la revendication 5, dans lequel lesdites plates-formes protéiques sont des puces protéiques.

7. Protéine selon la revendication 1, dans lequel ladite protéine est un marqueur biologique pour la détermination des profils immunologiques individuels.

8. Protéine selon la revendication 1, dans lequel ladite protéine est un marqueur biologique pour la discrimination entre les différents états de sclérose en plaques ; de préférence dans laquelle lesdits états sont choisis dans le groupe constitué par :
a. une forme récurrente-rémittente (RR) ;
b. une forme primaire ; et
c. une forme progressive secondaire.

9. Procédé de diagnostic de la sclérose en plaques à l'aide d'une protéine consistant en SEQ ID NO:2.

10. Procédé selon la revendication 9, comprenant les étapes consistant à :
a. fournir la protéine consistant en SEQ ID NO:2 immobilisée sur un substrat solide ;
b. mettre en contact un échantillon biologique obtenu précédemment avec le substrat de l'étape a.;
c. détecter la réaction.

11. Procédé selon la revendication 10, dans lequel ladite étape c. est effectuée au moyen d'une technique choisie dans le groupe comprenant :
a. des dosages de type ELISA ;
b. des dosages de type radioimmunologique (RIA) ;
c. des dosages de type immunologique (Western Blot et LINE blot) ;
d. des dosages de micro-réseaux de protéines.

12. Procédé selon l'une quelconque des revendications 9 à 11, pour discriminer entre des patients présentant des symptômes neurologiques et des patients atteints de sclérose en plaques.

13. Procédé selon la revendication 10, dans lequel ledit échantillon biologique est un sérum ou échantillon de liqueur.

14. Kit de diagnostic comprenant une protéine consistant en SEQ ID NO:2, qui sert d'antigène, et un réactif pour une utilisation séparée, simultanée et consécutive, pour le diagnostic de la sclérose en plaques.
